# EUROPEAN PATENT SPECIFICATION

(11) **EP 3 972 631 B1**
(45) Date of publication and mention of the grant of the patent: **10.07.2024**
(21) Application number: 20726457.3
(22) Date of filing: 20.05.2020
(51) Int. Cl.: A61K 38/46, A61P 43/00, A61P 25/00, A61K 48/00, C12N 9/00, C12N 15/00

(54) **EXPRESSION VECTOR FOR CHOLESTEROL 24-HYDROLASE IN THERAPY OF RETT SYNDROME**
EXPRESSIONSVEKTOR FÜR CHOLESTERIN-24-HYDROLASE IN DER THERAPIE DES RETT-SYNDROMS
VECTEUR D'EXPRESSION POUR LA CHOLESTÉROL 24-HYDROLASE DANS LE TRAITEMENT DU SYNDROME DE RETT

(30) Priority: 21.05.2019 EP 19175767
(43) Date of publication of application: 30.03.2022
(73) Proprietor: Institut National de la Santé et de la Recherche Médicale (INSERM), 75013 Paris (FR); CENTRE NATIONAL DE LA RECHERCHE SCIENTIFIQUE (CNRS), 75016 Paris (FR); Sorbonne Université, 75006 Paris (FR); ICM - Institut du Cerveau et de la Moelle Epinière, 75013 Paris (FR)
(72) Inventor: PIGUET, Françoise, 75013 Paris (FR); CARTIER-LACAVE, Nathalie, 75013 Paris (FR)
(74) Representative: Cabinet Becker et Associés
(86) International application number: PCT/EP2020/064092
(87) International publication number: WO 2020/234363

(56) References cited:
- EP-A1- 3 388 520
- WO-A1-2009/034127
- WO-A1-2012/049314
- WO-A1-2014/210389
- WO-A1-2016/049110
- WO-A1-2018/138371
- WO-A1-2018/226785
- KAMAL KE GADALLA ET AL: "Gene therapy for Rett syndrome: prospects and challenges", FUTURE NEUROLOGY, vol. 10, no. 5, 1 November 2015 (2015-11-01), GB, pages 467 - 484, XP055473364, ISSN: 1479-6708, DOI: 10.2217/fnl.15.29
- VASHI NEETI ET AL: "Treating Rett syndrome: from mouse models to human therapies", MAMMALIAN GENOME, SPRINGER NEW YORK LLC, US, vol. 30, no. 5, 28 February 2019 (2019-02-28), pages 90 - 110, XP036824827, ISSN: 0938-8990, [retrieved on 20190228], DOI: 10.1007/S00335-019-09793-5
- S. K. GARG ET AL: "Systemic Delivery of MeCP2 Rescues Behavioral and Cellular Deficits in Female Mouse Models of Rett Syndrome", THE JOURNAL OF NEUROSCIENCE, vol. 33, no. 34, 21 August 2013 (2013-08-21), US, pages 13612 - 13620, XP055475145, ISSN: 0270-6474, DOI: 10.1523/JNEUROSCI.1854-13.2013
- LOPEZ ADAM M ET AL: "Suppression of brain cholesterol synthesis in male Mecp2-deficient mice is age dependent and not accompanied by a concurrent change in the rate of fatty acid synthesis", BRAIN RESEARCH, ELSEVIER, AMSTERDAM, NL, vol. 1654, 24 October 2016 (2016-10-24), pages 77 - 84, XP029821662, ISSN: 0006-8993, DOI: 10.1016/J.BRAINRES.2016.10.021
- VILLANI CLAUDIA ET AL: "Lovastatin fails to improve motor performance and survival in methyl-CpG-binding protein2-null mice", ELIFE, ELIFE SCIENCES PUBLICATIONS LTD, GB, vol. 5, 28 November 2016 (2016-11-28), pages 1, XP009530387, ISSN: 2050-084X, DOI: 10.7554/ELIFE.22409
- DIETER LÜTJOHANN ET AL: "Identification of Correlative Shifts in Indices of Brain Cholesterol Metabolism in the C57BL6/ Mecp2 tm1.1Bird Mouse, a Model for Rett Syndrome", LIPIDS, vol. 53, no. 4, 1 April 2018 (2018-04-01), DE, pages 363 - 373, XP055633792, ISSN: 0024-4201, DOI: 10.1002/lipd.12041

## Description

The present invention is defined in claims 1-14 and relates to the treatment of Rett Syndrome.

### BACKGROUND OF THE INVENTION

Rett syndrome (RTT) is a neurodevelopmental disorder that affects girls almost exclusively (about 1 in 10 000 females). It is characterized by normal early growth and development followed by a slowing of development, loss of purposeful use of the hands, distinctive hand movements, slowed brain and head growth, problems with walking, seizures, and intellectual disability (Hagberg et al. 1985).

The course of Rett syndrome, including the age of onset and the severity of symptoms, varies from child to child. Before the symptoms begin, however, the child generally appears to grow and develop normally, although there are often subtle abnormalities even in early infancy, such as loss of muscle tone (hypotonia), difficulty feeding, and jerkiness in limb movements. Then, gradually, mental and physical symptoms appear. As the syndrome progresses, the child loses purposeful use of her hands and the ability to speak. Other early symptoms may include problems crawling or walking and diminished eye contact. The loss of functional use of the hands is followed by compulsive hand movements such as wringing and washing. The onset of this period of regression is sometimes sudden (Armstrong 1997).

Children with Rett syndrome often exhibit autistic-like behaviors in the early stages. Other symptoms may include walking on the toes, sleep problems, a wide-based gait, teeth grinding and difficulty chewing, slowed growth, seizures, cognitive disabilities, and breathing difficulties while awake such as hyperventilation, apnea (breath holding), and air swallowing. Rett syndrome is caused by mutation in the X-linked gene methyl-CpG-binding protein 2 (MECP2), a ubiquitously expressed transcriptional regulator (Amir et al. 1999; Duncan Armstrong 2005).

Mouse models recapitulate many of the symptoms of RTT and their study has provided insight into the physiological basis of disease. Although female *Mecp2*/+ mice show phenotypic variance in part due to random X-chromosome inactivation, male *Mecp2*/Y mice have a fully penetrant phenotype. Null males are normal at birth and weaning, but develop limb clasping, tremors, lethargy and abnormal breathing, symptoms that progressively worsen until death at 6 - 16 weeks of age (Guy et al. 2001).

In animal models of RTT disease, several studies already demonstrated that loss of *Mecp2* disrupts cholesterol homeostasis. Indeed, genes in the mevalonate pathway were slightly upregulated in the brains, but not livers in 1-month-old *Mecp2*/Y mice (mild affection). At 2 months of age, total cholesterol concentrations were increase in the brains of symptomatic mutant mice (Nagy and Ackerman 2013). It has also been reported in MECP2 deficient males that Cyp46a1 expression is increased 38% over wild-type levels, indicating a heightened need for cholesterol turnover in neurons (Buchovecky et al. 2013; Lütjohann et al. 2018a).

A therapeutic approach has also been tried by administrating Statins, which inhibit 3-hydroxy-3-methylglutaryl-coenzyme A (HMG-CoA) reductase (HMGCR), the first rate-limiting enzyme in cholesterol synthesis. Statin treatment enhanced the median survival time of the mutant male mice and improved motor coordination and activity, but did not improve breathing irregularities and acoustic startle response abnormalities. To more closely model treatment of female patients with RTT, *Mecp2*/+ female mice were also treated with statins, which ameliorated symptoms (Buchovecky et al. 2013).

Up to now, there is no known cure for Rett syndrome. The only molecules that are currently used only aim at decreasing the secondary effects associated with Rett syndrome. Medication may be needed for breathing irregularities and motor difficulties, and anticonvulsant drugs may be used to control seizures. There should be regular monitoring for scoliosis and possible heart abnormalities. However, several approaches are actually under evaluation in mouse models of the disease. They mainly aim at restoring MECP2 expression using gene therapy delivery (Garg et al. 2013)(K. K. Gadalla et al. 2013; K. K. E. Gadalla et al. 2017), leading to an increase survival, but without necessary improving the phenotype notably coordination performances. In addition, a hepatic toxicity has been highlighted when deliver at high dose. MECP2 is a transcription factor, which expression is strongly regulated to maintain a synaptic regulation and neuronal homeostasis. Thus, increasing MECP2 levels (by 2 fold the endogenous level) lead to a Rett-like disorder with opposite phenotype of MECP2 loss of function (Na et al. 2013). There is thus an acute need to develop new strategies for therapy for Rett patients.

### SUMMARY OF THE INVENTION

The inventors now propose to counteract Rett syndrome by modulating the cholesterol metabolism pathway, more specifically by means of a vector comprising cholesterol 24-hydroxylase encoding nucleic acid that expresses cholesterol 24-hydroxylase in the target cells. It is therefore an object of the present invention to provide a vector for use in the treatment of Rett syndrome, which vector comprises cholesterol 24-hydroxylase encoding nucleic acid.

In an embodiment, the vector comprises a nucleic acid sequence that encodes the amino acid sequence SEQ ID N°2. Alternatively, the vector comprises the nucleic acid sequence SEQ ID N°1.

In an embodiment, the vector is selected from the group of adenovirus, lentivirus, retrovirus, herpes-virus and Adeno-Associated Virus (AAV) vectors, preferably an AAV vector, more preferably an AAV9, AAV10 (AAVrh. 10) or AAVPHP.eB vector, even more preferably an AAVPHP. eB.

In an embodiment, the vector is administered directly into the brain of the patient, preferably to the brain in all regions of interest (hippocampus, cortex, striatum, pons and cerebellum). In an embodiment, the vector is administered by intravascular, intravenous, intranasal, intraventricular, intrathecal or retro-orbital injection. In a particular embodiment, the vector is administered intravenously. In a particular embodiment, the vector is administered in the cerebrospinal fluid.

It is another object of the invention to provide a pharmaceutical composition for use in the treatment of Rett syndrome, which comprises a vector comprising cholesterol 24-hydroxylase encoding nucleic acid.

### FIGURES

*Figure* 1- **Basal levels of CYP46A1 in brain areas** (the cerebellum, pons, striatum, hippocampus and cortex) of aggravated MECP2 KO male mouse model at 45 days, compared to wild-type (control) littermates (WT).
*Figure* 2- **Lipid abnormalities in the brain of aggravated MECP2 KO males** at 45 days compared to wild-type (control) littermates (WT).
*Figure* 3- **Behavioral evaluation of mild MECP2 KO male mice** after intravenous administration of AAVPHP.eB- CYP46A1 (KO MECP2 AAV) at 3 weeks compared to non-treated male MECP2 KO mice (KO MECP2) and control (WT). (A) Clasping test; (B) Rotarod; (C) Weight follow up. Results are presented as mean ± SEM.
*Figure* 4 - **Biodistribution of AAVPHP.eB-CYP46A1** in central nervous system (A) and peripheral organs (B) of mild male KO MECP2.
*Figure 5* - **Evaluation of target engagement through 24OH cholesterol levels quantification at 16 weeks of age in mild KO MECP2 males.** Brain 24S-hydroxycholesterol content in mild KO MECP2 male mice assessed by UPLC-HRMS. Results are presented as mean ± SEM.
*Figure* 6 - **Evaluation at 16 weeks of Purkinje cells and histological analysis after intravenous delivery of AAVPHP.eB-CYP46A1 in mild KO MECP2 males.** (Quantification of Purkinje cell numbers in cerebellum. Results are presented as mean ± SEM).
*Figure* 7- **Evaluation at 16 weeks of astrogliosis and histological analysis after intravenous delivery of AAVPHP.eB-CYP46A1 as treatment in mild KO MECP2 males.** Quantification of astrocytes numbers in different regions of brain. Results are presented as mean ± SEM.
*Figure 8* - **Evaluation at 16 weeks of microgliosis and histological analysis after intravenous delivery of AAVPHP.eB-CYP46A1 as treatment in KO MECP2 mild male mice.** Quantification of microglial cell numbers in different regions of brain. Results are presented as mean ± SEM.
*Figure* 9 - **Behavioral evaluation of aggravated MECP2 KO male mice** after intravenous administration of AAVPHP.eB- CYP46A1 (KO MECP2 AAV) at 3 weeks compared to non-treated male MECP2 KO mice (KO MECP2) and control (WT). (A) Clasping test; (B) Rotarod; (C) Weight follow up. Results are presented as mean ± SEM.
*Figure 10* - **Biodistribution of AAVPHP.eB-CYP46A1** in central nervous system (A) and peripheral organs (B) of aggravated KO MECP2 males at 40 days.
*Figure 11* - **mRNA levels of genes involved in cholesterol metabolism at 6 weeks in lumbar brain of aggravated KO MECP2 males.** mRNA was extracted from the total brain of 6 week-old mice and murine and human (A) CYP46A1 levels and other genes involved in cholesterol pathway (B) were quantified. mRNA levels were normalized to actin housekeeping gene. Data are represented as mean ± SEM (n = 6 per group). Data are represented as mean ± SEM (n = 7-9 per group). Statistical analysis: student t-test. *p<0.05, **p<0.01.
*Figure* 12 - **Evaluation at 6 weeks of Purkinje cells and histological analysis after intravenous delivery of AAVPHP.eB-CYP46A1 as treatment in aggravated KO MECP2 males.** Quantification of Purkinje cell numbers in cerebellum. Results are presented as mean ± SEM.
*Figure* 13 - **Evaluation at 6 weeks of astrogliosis and histological analysis after intravenous delivery of AAVPHP.eB-CYP46A1 as treatment in aggravated KO MECP2 males.** Quantification of astrocytes numbers in different regions of brain. Results are presented as mean ± SEM.
*Figure 14* - **Evaluation at 16 weeks of microgliosis and histological analysis after intravenous delivery of AAVPHP.eB-CYP46A1 as treatment in aggravated KO MECP2 male mice.** Quantification of microglial cell numbers in different regions of brain. Results are presented as mean ± SEM.
*Figure 15* - **Behavioral evaluation of aggravated MECP2 KO female mice** after intravenous administration of AAVPHP.eB- CYP46A1 (KO MECP2 AAV) at 12 weeks compared to non-treated male MECP2 KO mice (KO MECP2) and control (WT). (A) Clasping test; (B) Rotarod; (C) Weight follow up. Results are presented as mean ± SEM.

### DETAILED DESCRIPTION OF THE INVENTION

The inventors demonstrated that delivering a vector expressing a CYP46A1 gene intravenously in a mouse model of RTT is able to prevent/correct the development of motor impairment, in a mild and an aggravated model of the disease both in male and female mice. In addition in males, the inventors demonstrated a prevention of the loss of Purkinje cells and an improvement of astrogliosis and microgliosis in the mild KO MECP2 model.

On this basis, the inventors provide a viral vector for the treatment of Rett syndrome, wherein the vector expresses CYP46A1 in cells of the central nervous system, especially neurons within cortex, hippocampus, striatum and cerebellum. This strategy could be useful in treating other pathology with intellectual disabilities or autism spectrum disorders related to Rett syndrome.

### Rett Syndrome

The present invention specifically relates to treatment of Rett syndrome. Preferably, the present invention relates to treatment of Rett syndrome which is caused by mutation in the X-linked gene methyl-CpG-binding protein 2 (MECP2), a ubiquitously expressed transcriptional regulator. In an embodiment, the present invention relates to treatment of Rett syndrome associated with intellectual disabilities.

In the context of the invention, the terms "treatment", "treat" or "treating" are used herein to characterize a therapeutic method or process that is aimed at (1) slowing down or stopping the progression, aggravation, or deterioration of the symptoms of the disease state or condition to which such term applies; (2) alleviating or bringing about ameliorations of the symptoms of the disease state or condition to which such term applies; and/or (3) reversing or curing the disease state or condition to which such term applies.

As used herein, the term "subject" or "patient" refers to an animal, preferably to a mammal, even more preferably to a human, including adult and child. However, the term "subject" can also refer to non-human animals, mammals such as mouse, and non-human primates.

### The CYP46A1 sequences

A first object of the invention relates to a vector for use in the treatment of Rett syndrome, which comprises the full sequence of cholesterol 24-hydroxylase encoding nucleic acid.

As used herein, the term "gene" refers to a polynucleotide containing at least one open reading frame that can encode a particular polypeptide or protein after being transcribed or translated. As used herein, the terms "coding sequence" or "a sequence which encodes a particular protein", denotes a nucleic acid sequence which is transcribed (in the case of DNA) and translated (in the case of mRNA) into a polypeptide in vitro or in vivo when placed under the control of appropriate regulatory sequences. The boundaries of the coding sequence are determined by a start codon at the 5' (amino) terminus and a translation stop codon at the 3' (carboxy) terminus. A coding sequence can include, but is not limited to, cDNA from prokaryotic or eukaryotic mRNA, genomic DNA sequences from prokaryotic or eukaryotic DNA, and even synthetic DNA sequences.

The CYP46A1 gene encodes cholesterol 24-hydroxylase. This enzyme is a member of the cytochrome P450 superfamily of enzymes. The enzyme converts cholesterol into 24S-hydroxycholesterol (24S-OH-Chol) that can dynamically cross the BBB, accomplishing peripheral circulation to be evacuated out of the body (Björkhem, I. et al.1998), thus maintaining cholesterol homeostasis. A cDNA sequence for CYP46A1 is disclosed in Genbank Access Number AF094480 (SEQ ID NO:1). The amino acid sequence is shown in SEQ ID NO:2.

The invention makes use of a nucleic acid construct comprising sequence SEQ ID NO:1 or a variant thereof for the treatment of Rett syndrome, and optionally related autism spectrum related disorders.

Variants exhibit a nucleotide sequence identity of at least 75%, preferably at least 85%, more preferably at least 90%, more preferably at least 95% with SEQ ID NO: 1.

### Non-viral vectors

In an embodiment, the vector use according to the present invention is a non-viral vector. Typically, the non-viral vector may be a plasmid encoding CYP46A1. This plasmid can be administered directly or through a liposome, an exosome or a nanoparticle.

### Viral vectors

Gene delivery viral vectors useful in the practice of the present invention can be constructed utilizing methodologies well known in the art of molecular biology. Typically, viral vectors carrying transgenes are assembled from polynucleotides encoding the transgene, suitable regulatory elements and elements necessary for production of viral proteins which mediate cell transduction.

The terms "gene transfer" or "gene delivery" refer to methods or systems for reliably inserting foreign DNA into host cells. Such methods can result in transient expression of non-integrated transferred DNA, extrachromosomal replication and expression of transferred replicons (e. g., episomes), or integration of transferred genetic material into the genomic DNA of host cells. Examples of viral vector include adenovirus, lentivirus, retrovirus, herpes-virus and Adeno-Associated virus (AAV) vectors.

Such recombinant viruses may be produced by techniques known in the art, such as by transfecting packaging cells or by transient transfection with helper plasmids or viruses. Typical examples of virus packaging cells include PA317 cells, PsiCRIP cells, GPenv+ cells, 293 cells, etc. Detailed protocols for producing such replication-defective recombinant viruses may be found for instance in WO95/14785, WO96/22378, US5,882,877, US6,013,516, US4,861,719, US5,278,056 and WO94/19478.

In a preferred embodiment, adeno-associated viral (AAV) vectors are employed.

By an "AAV vector" is meant a vector derived from an adeno-associated virus serotype, including without limitation, AAV1, AAV2, AAV3, AAV4, AAV5, AAV6, AAV8, AAV9, AAV 10, such as AAVrh. 10, AAVPHP. eB, etc. AAV vectors can have one or more of the AAV wild-type genes deleted in whole or part, preferably the rep and/or cap genes, but retain functional flanking ITR sequences. Functional ITR sequences are necessary for the rescue, replication and packaging of the AAV virion. Thus, an AAV vector is defined herein to include at least those sequences required in cis for replication and packaging (e. g., functional ITRs) of the virus. The ITRs need not be the wild-type nucleotide sequences, and may be altered, e. g , by the insertion, deletion or substitution of nucleotides, so long as the sequences provide for functional rescue, replication and packaging. AAV expression vectors are constructed using known techniques to at least provide as operatively linked components in the direction of transcription, control elements including a transcriptional initiation region, the DNA of interest (i.e. the CYP46A1 gene) and a transcriptional termination region. Two copies of the DNA of interest can be included, as a self-complementary construct14.

In a more preferred embodiment, the AAV vector is an AAV9, AAV10 (AAVrh. 10), AAVPHP.B or AAVPHP.eB vector, or vector derived from one of these serotypes. In a most preferred embodiment, the AAV vector is an AAVPHP.eB vector. The AAVPHP.eB vector is evolved AAV-PHP.B variant that efficiently transduces CNS neurons (Ken Y Chan et al. 2017; WO2017100671). Other vectors, such as the ones described in WO2015038958 and WO2015191508 may also be used.

The control elements are selected to be functional in a mammalian cell. The resulting construct which contains the operatively linked components is bounded (5' and 3') with functional AAV ITR sequences. By "adeno-associated virus inverted terminal repeats " or "AAV ITRs" is meant the art-recognized regions found at each end of the AAV genome which function together in cis as origins of DNA replication and as packaging signals for the virus. AAV ITRs, together with the AAV rep coding region, provide for the efficient excision and rescue from, and integration of a nucleotide sequence interposed between two flanking ITRs into a mammalian cell genome. The nucleotide sequences of AAV ITR regions are known. (See, e. g., Kotin, 1994; Berns, KI "Parvoviridae and their Replication" in Fundamental Virology, 2nd Edition, (B. N. Fields and D. M. Knipe, eds.) for the AAV-2 sequence. As used herein, an "AAV ITR" does not necessarily comprise the wild-type nucleotide sequence, but may be altered, e. g., by the insertion, deletion or substitution of nucleotides. Additionally, the AAV ITR may be derived from any of several AAV serotypes, including without limitation, AAV-1, AAV-2, AAV-3, AAV-4, AAV-5, AAV6, etc. Furthermore, 5' and 3' ITRs which flank a selected nucleotide sequence in an AAV vector need not necessarily be identical or derived from the same AAV serotype or isolate, so long as they function as intended, i.e., to allow for excision and rescue of the sequence of interest from a host cell genome or vector, and to allow integration of the heterologous sequence into the recipient cell genome when AAV Rep gene products are present in the cell.

Particularly preferred are vectors derived from AAV serotypes having tropism for and high transduction efficiencies in cells of the mammalian central nervous system (CNS), particularly neurons. A review and comparison of transduction efficiencies of different serotypes is provided in Davidson et al., 2000. In one preferred example, AAV2 based vectors have been shown to direct long-term expression of transgenes in CNS, preferably transducing neurons. In other non-limiting examples, preferred vectors include vectors derived from AAV4 and AAV5 serotypes, which have also been shown to transduce cells of the CNS (Davidson et al, supra). In particular, the vector may be an AAV vector comprising a genome derived from AAV5 (in particular the ITRs are AAV5 ITRs) and a capsid derived from AAV5.

In a particular embodiment of the invention, the vector is a pseudotyped AAV vector. Specifically, a pseudotyped AAV vector comprises an AAV genome derived from a first AAV serotype and a capsid derived from a second AAV serotype. Preferably, the genome of the AAV vector is derived from AAV2. Furthermore, the capsid is preferably derived from AAV5. Specific non-limiting examples of pseudotyped AAV vectors include an AAV vector comprising a genome derived from AAV2 in a capsid derived from AAV5, an AAV vector comprising a genome derived from AAV2 in a capsid derived from AAVrh. 10, etc.

The selected nucleotide sequence is operably linked to control elements that direct the transcription or expression thereof in the subject in vivo. Such control elements can comprise control sequences normally associated with the selected gene. In particular, such control elements may include the promoter of the CYP46A1 gene, in particular the promoter of the human CYP46A1 gene (Ohyama Y et al., 2006)

Alternatively, heterologous control sequences can be employed. Useful heterologous control sequences generally include those derived from sequences encoding mammalian or viral genes. Examples include, but are not limited to, the phophoglycerate kinase (PGK) promoter, the SV40 early promoter, mouse mammary tumor virus LTR promoter; adenovirus major late promoter (Ad MLP); a herpes simplex virus (HSV) promoter, a cytomegalovirus (CMV) promoter such as the CMV immediate early promoter region (CMVIE), rous sarcoma virus (RSV) promoter, synthetic promoters, hybrid promoters, and the like. In addition, sequences derived from nonviral genes, such as the murine metallothionein gene, will also find use herein. Such promoter sequences are commercially available from, e. g., Stratagene (San Diego, CA). For purposes of the present invention, both heterologous promoters and other control elements, such as CNS-specific and inducible promoters, enhancers and the like, will be of particular use. Examples of heterologous promoters include the CMV promoter. Examples of CNS specific promoters include those isolated from the genes from myelin basic protein (MBP), glial fibrillary acid protein (GFAP), synapsins (e.g. human synapsin 1 gene promoter), and neuron specific enolase (NSE).

Examples of inducible promoters include DNA responsive elements for ecdysone, tetracycline, hypoxia andaufin.

The AAV expression vector which harbors the DNA molecule of interest bounded by AAV ITRs, can be constructed by directly inserting the selected sequence(s) into an AAV genome which has had the major AAV open reading frames ("ORFs") excised therefrom. Other portions of the AAV genome can also be deleted, so long as a sufficient portion of the ITRs remain to allow for replication and packaging functions. Such constructs can be designed using techniques well known in the art. See, e. g., U. S. Patents Nos. 5,173,414 and 5,139,941; International Publications Nos. WO 92/01070 (published 23 January 1992) and WO 93/03769 (published 4 March 1993); Lebkowski et al., 1988 ; Vincent et al., 1990; Carter, 1992 ; Muzyczka, 1992 ; Kotin, 1994; Shelling and Smith, 1994 ; and Zhou et al., 1994. Alternatively, AAV ITRs can be excised from the viral genome or from an AAV vector containing the same and fused 5' and 3' of a selected nucleic acid construct that is present in another vector using standard ligation techniques. AAV vectors which contain ITRs have been described in, e. g., US. Patent no. 5,139,941. In particular, several AAV vectors are described therein which are available from the American Type Culture Collection ("ATCC") under Accession Numbers 53222, 53223, 53224, 53225 and 53226. Additionally, chimeric genes can be produced synthetically to include AAV ITR sequences arranged 5' and 3' of one or more selected nucleic acid sequences. Preferred codons for expression of the chimeric gene sequence in mammalian CNS cells can be used. The complete chimeric sequence is assembled from overlapping oligonucleotides prepared by standard methods. (See, e. g., Edge, 1981 ; Nambair et al., 1984 ; Jay et al., 1984). In order to produce rAAV virions, an AAV expression vector is introduced into a suitable host cell using known techniques, such as by transfection. A number of transfection techniques are generally known in the art. (See, e.g., Graham et al., 1973; Sambrook et al. (1989) Molecular Cloning, a laboratory manual, Cold Spring Harbor Laboratories, New York, Davis et al. (1986) Basic Methods in Molecular Biology, Elsevier, and Chu et al., 1981). Particularly suitable transfection methods include calcium phosphate coprecipitation (Graham et al., 1973), direct microinjection into cultured cells (Capecchi, 1980), electroporation (Shigekawa et al., 1988), liposome mediated gene transfer (Mannino et al., 1988), lipid-mediated transduction (Felgner et al., 1987), and nucleic acid delivery using high-velocity microprojectiles (Klein et al., 1987).

For instance, a preferred viral vector, such as the AAVPHP.eB, comprises, in addition to a cholesterol 24-hydroxylase encoding nucleic acid sequence, the backbone of AAV vector with ITR derived from AAV-2, the promoter, such as the mouse PGK (phosphoglycerate kinase) gene or the cytomegalovirus/β-actin hybrid promoter (CAG) consisting of the enhancer from the cytomegalovirus immediate gene, the promoter, splice donor and intron from the chicken β-actin gene, the splice acceptor from rabbit β-globin, or any neuronal promoter such as the promoter of Dopamine-1 receptor or Dopamine-2 receptor with or without the wild-type or mutant form of woodchuck hepatitis virus post-transcriptional regulatory element (WPRE).

### Delivery of the vectors

A method of treatment of Rett syndrome is disclosed, which method comprises administering a vector comprising cholesterol 24-hydroxylase encoding nucleic acid to a patient in need thereof. The vector may be delivered directly into the brain and/or spinal cord of the subject or by intravascular, intravenous, retroorbital, intranasal, intraventricular or intrathecal injection.

In a particular embodiment, it is provided a method for treating Rett syndrome (RTT) in a subject, said method comprising:
(a) providing a vector as defined above, which comprises a cholesterol 24-hydroxylase encoding nucleic acid; and
(b) delivering the vector to the brain and/or spinal cord of the subject, whereby said vector transduces cells in the brain and/or spinal cord, and whereby cholesterol 24-hydroxylase is expressed by the transduced cells at a therapeutically effective level.

Advantageously, the vector is a viral vector, more advantageously an AAV vector, even advantageously an AAV vector selected from the group consisting of AAV 9, AAV10, or AAVPHP.eB, preferably AAVPHP.eB.

In a particular embodiment, the vector is delivered to brain, particularly to motor cortex, and/or spinal cord. In a particular embodiment, the vector is delivered exclusively to spinal cord.

Methods of delivery, or administration, of viral vectors to neurons and/or astrocytes and/or oligodendrocytes and/or microglia include generally any method suitable for delivery vectors to said cells, directly or through hematopoietic cells transduction, such that at least a portion of cells of a selected synaptically connected cell population is transduced. The vector may be delivered to any cells of the central nervous system, cells of the peripheral nervous system, or both. Preferably, the vector is delivered to cells of the brain and/or spinal cord. Generally, the vector is delivered to the cells of the brain, including for example cells of motor cortex, spinal cord or combinations thereof, or any suitable subpopulation thereof.

To deliver the vector specifically to a particular region and to a particular population of cells of the brain or the spinal cord, the vector may be administered by stereotaxic microinjection. For example, patients have the stereotactic frame base fixed in place (screwed into the skull). The brain with stereotactic frame base (MRI compatible with fiducial markings) is imaged using high resolution MRI. The MRI images are then transferred to a computer which runs stereotactic software. A series of coronal, sagittal and axial images are used to determine the target (site of vector injection) and trajectory. The software directly translates the trajectory into 3 dimensional coordinates appropriate for the stereotactic frame. Burr holes are drilled above the entry site and the stereotactic apparatus positioned with the needle implanted at the given depth. The vector is then injected at the target sites, eventually mixed with a contrast agent. Since the vector integrates into the target cells, rather than producing viral particles, the subsequent spread of the vector is minor, and mainly a function of passive diffusion from the site of injection and of course the desired trans-synaptic transport, prior to integration. The degree of diffusion may be controlled by adjusting the ratio of vector to fluid carrier. Additional routes of administration may also comprise local application of the vector under direct visualization, e. g., superficial cortical application, intranasal application, or other non-stereotactic application.

The target cells of the vectors of the present invention are cells of the brain (neurons) in the hippocampus, cortex, striatum and cerebellum of a subject afflicted with RTT, preferably neural cells. Preferably, the subject is a human being, generally a child or an infant, and particularly a female infant, but may be an adult.

However, the invention encompasses delivering the vector to biological models of the disease. In that case, the biological model may be any mammal at any stage of development at the time of delivery, e.g., embryonic, foetal, infantile, juvenile or adult, preferably it is an adult. Furthermore, the target cells may be essentially from any source, especially nonhuman primates and mammals of the orders Rodenta (mice, rats, rabbit, hamsters), Carnivora (cats, dogs), and Arteriodactyla (cows, pigs, sheep, goats, horses) as well as any other non-human system (e.g. zebrafish model system).

Preferably, the method of the invention comprises intracerebral administration, through stereotaxic injections. However, other known delivery methods may also be adapted in accordance with the invention. For example, for a more widespread distribution of the vector across the brain, it may be injected into the cerebrospinal fluid, e.g., by lumbar puncture, cisterna magna or ventricular puncture. To direct the vector to the brain, it may be injected into the spinal cord or into the peripheral ganglia, or the flesh (subcutaneously or intramuscularly) of the body part of interest. In certain situations, the vector, such as here with AAVPHP.eB can be administered via an intravascular approach. For example, the vector can be administered intra-arterially (carotid) in situations where the blood-brain barrier is disturbed. Moreover, for more global delivery, the vector can be administered during the "opening" of the blood-brain barrier achieved by infusion of hypertonic solutions including mannitol or ultra-sound local delivery.

The vectors used herein may be formulated in any suitable vehicle for delivery. For instance, they may be placed into a pharmaceutically acceptable suspension, solution or emulsion. Suitable mediums include saline and liposomal preparations. More specifically, pharmaceutically acceptable carriers may include sterile aqueous of non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Intravenous vehicles include fluid and nutrient replenishers, electrolyte replenishers (such as those based on Ringer's dextrose), and the like. Preservatives and other additives may also be present such as, for example, antimicrobials, antioxidants, chelating agents, and inert gases and the like.

A colloidal dispersion system may also be used for targeted gene delivery. Colloidal dispersion systems include macromolecule complexes, nanocapsules, microspheres, beads, and lipid-based systems including oil-in-water emulsions, micelles, mixed micelles, and liposomes or exosomes.

The preferred doses and regimen may be determined by a physician, and depend on the age, sex, weight, of the subject, and the stage of the disease. As an example, for delivery of cholesterol 24-hydroxylase using a viral expression vector, each unit dosage of cholesterol 24-hydroxylase expressing vector may comprise 2.5 to 1 ml of a composition including a viral expression vector in a pharmaceutically acceptable fluid and which provides from 1010 up to 1015 cholesterol 24-hydroxylase expressing viral particles per ml of composition.

### Pharmaceutical composition

A further object of the invention concerns a pharmaceutical composition for use in the treatment of Rett syndrome, which comprises a therapeutically effective amount of a vector according to the invention.

By a "therapeutically effective amount" is meant a sufficient amount of the vector of the invention to treat Rett syndrome at a reasonable benefit/risk ratio applicable to any medical treatment.

It will be understood that the total daily dosage of the compounds and compositions of the present invention will be decided by the attending physician within the scope of sound medical judgment. The specific therapeutically effective dose level for any particular patient will depend upon a variety of factors including the disorder being treated and the severity of the disorder; activity of the specific compound employed; the specific composition employed, the age, body weight, general health, sex and diet of the patient; the time of administration, route of administration, and rate of excretion of the specific compound employed; the duration of the treatment; drugs used in combination or coincidental with the specific polypeptide employed; and like factors well known in the medical arts. For example, it is well within the skill of the art to start doses of the compound at levels lower than those required to achieve the desired therapeutic effect and to gradually increase the dosage until the desired effect is achieved. However, the daily dosage of the products may be varied over a wide range per adult per day. The therapeutically effective amount of the vector according to the invention that should be administered, as well as the dosage for the treatment of a pathological condition with the number of viral or non-viral particles and/or pharmaceutical compositions of the invention, will depend on numerous factors, including the age and condition of the patient, the severity of the disturbance or disorder, the method and frequency of administration and the particular peptide to be used.

The presentation of the pharmaceutical compositions that contain the vector according to the invention may be in any form that is suitable for intramuscular, intracerebral, intranasal, intrathecal, intraventricular or intravenous administration. In the pharmaceutical compositions of the present invention for intramuscular, intranasal, retroorbital, intravenous, intracerebral, intrathecal or intraventricular administration, the active principle, alone or in combination with another active principle, can be administered in a unit administration form, as a mixture with conventional pharmaceutical supports, to animals and human beings.

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of sterile injectable solutions.

Upon formulation, solutions will be administered in a manner compatible with the dosage formulation and in such amount as is therapeutically effective. The formulations are easily administered in a variety of dosage forms, such as the type of injectable solutions, but drug release capsules and the like can also be employed.

Multiple doses can also be administered.

It is also possible to associate the therapeutical approach with small molecules that could activate CYP46A1 such as efavirenz (Mast, N. et al. 2017; Mast, N. et al. 2014) or antifungal drugs (Mast, N. et al. 2013; Fourgeux, C. et al. 2014), which on the contrary could inhibit CYP46A1 and thus be a way to stop the gene therapy approach if needed.

The invention will be further illustrated by the following example. However, this example and the accompanying figures should not be interpreted in any way as limiting the scope of the present invention.

### EXAMPLE

In this experiment, a severe mouse model of Rett syndrome has been used and a significant rescue of the phenotype has been demonstrated.

### Materials and Methods

### Animals

Two lines of mice have been used in this study.

Three females B6.129P2-*Mecp2^{tm1Bird}*/J homozygous were obtained from Jackson Laboratories (stock 007177) and mated with C57BL/6 mice to generate the colony, named thereafter mild MECP2 KO.

Ten females B6.129P2(C)-*Mecp2^{tm.11Bird}*/J heterozygous were obtained from Jackson Laboratories (stock 003890) and mated with C57BL/6 mice to generate the colony, named thereafter aggravated MECP2 KO.

Mice were housed in a temperature-controlled room and maintained on a 12 h light/dark cycle. Food and water were available ad libitum. The experiments were carried out in accordance with the European Community Council directive (2010/63/EU) for the care and use of laboratory animals.

### AAV plasmid design and vector production

AAV vectors were produced and purified by Atlantic Gene therapies (INSERM U1089, Nantes, France). Vector production has been described elsewhere (Hudry et al., 2010). The viral constructs for AAVPHP.eB-CYP46A1-HA contained the expression cassette consisting of the human Cyp46a1gene, driven by a CMV early enhancer/chicken β-actin (CAG) synthetic promoter (CAG) surrounded by inverted terminal repeats (ITR) sequences of AAV2.

### MECP2 genotyping

Mice were genotype according to the Jackson lab procedure (https://www2.jax.org/protocolsdb/f?p=116:5:0::NO:5:P5_MASTER_PROTOCOL_ID,P5_J RS CODE:28595,007177) and (https://www.iax.org/Protocol?stockNumber=003890&protocolID=24870) depending on the colony.

### Intravenous injection

Three-week-old MECP2^{-/y} male (mild and aggravated) mice and 12 weeks old MECP2 +/aggravated females were induced for anesthesia with isoflurane at 4% and then maintained at 2% with 80% air and 20% oxygen. Mild MECP2^{-/y} animal (n=14) received an injection of 5.10e11vg total (100ul volume) intravenously by retroorbital injection. WT and non-injected aggravated MECP2^{-/y} animals (n=10-13 per group respectively) received injection of 100µl of saline solution. MECP2^{-/y} animal (n=11) received an injection of 5.10e11vg total (100µl volume) intravenously by retroorbital injection. WT and non-injected MECP2^{-/y} animals (n=17-19 per group respectively) received injection of 100µl of saline solution.

Aggravated MECP2^{+/-} female animals (n=8) received an injection of 5.10e11vg total (100µl volume) intravenously by retroorbital injection. WT and non-injected MECP2^{+/-} animals (n=9-10 per group respectively) received injection of 100µl of saline solution.

### Behavioral tests

### Clasping test

Clasping test evaluates coordination and is classical for Rett evaluation. Animals were scored prior to injection and then each week from 3 weeks until 32 weeks (for mild KO males) until 6 weeks for aggravated KO males. For aggravated females, they were scored 1 week after injection and then each 3 weeks (until 25 weeks for now but ongoing). Animals are maintained from the tail and the score is "1" if mice are twitching, and then a point is added to the score for each hindlimbs clasping. Results are presented for each test as mean ± SEM for each group and 2 way ANOVA analyses were performed.

### Rotarod

General motor capacities were tested using the accelerating rotarod LE8200 (Bioseb, France) and were measured as previously described (https://www.mousephenotype.org/). Animals were scored weekly from 3 weeks of age until maximal survival (6 weeks for aggravated KO males, 28 weeks for mild KO males and up to now 19 weeks for aggravated KO females).

### Tissue collection

Mice were anesthetized with pentobarbital (Euthasol 180 mg/kg) solution and perfused transcardially with phosphate buffered saline (PBS). Brain, spinal cord and sciatic nerves as well as peripheral organs (liver, heart, lung, kidney, spleen, diaphragm, gastrocnemium) were collected and post-fixed in PFA 4% prior to paraffin inclusion for histology (Cut 6-10 µm with microtome) or immediately frozen in liquid nitrogen for biomolecular analysis.

Different tissues were grinded/crushed in liquid azote and were separated to analyze RNA, DNA expression or lipidomic analysis.

### Primary antibodies

Antibodies used immunohistochemical (IHC) analyses are listed in Table 1, below.

**Table 1: Antibodies used in immunohistochemical (IHC) analyses**

| **Primary antibodies** | **Source** | **IHC** |
|---|---|---|
| Rabbit anti-Calbindin D-28k | Swant (CB38a) | 1:10000 |
| Rabbit anti-HA | Cell signaling (#3724) | 1:500 |
| Rabbit anti-Iba1 | Wako (019-19741) | 1:500 |
| Mouse anti-GFAP | Sigma (G-3893) | 1:500 |

### Immunostaining

### On paraffin sections

The immunohistochemical labeling was performed using the ABC method. Briefly, tissue sections were treated with peroxide for 30 minutes to inhibit endogenous peroxidase. After washes in PBS, sections were treated 10 mM Tris/1 mM EDTA/0.1% Tween pH8.75 at 95°C for 45 min (Only for anti-HA) or citrate 10 mM pH6 at 95°C for 45 min (Only for anti-CB) . After washes in PBS, sections were incubated with the blocking solution (10% goat serum in PBS/0,3% TritonX-100) for 1 hour. The primary antibodies were diluted in blocking solution and incubated on tissue sections overnight at 4°C. After washes in PBS, sections were sequentially incubated with goat anti-rabbit or goat anti-mouse antibodies conjugated to biotin (Vector Laboratories) for 30 minutes at room temperature, followed by the ABC complex (Vector Laboratories). After washes in PBS, the peroxidase activity was detected using diaminobenzidine as chromogene (Dako, Carpinteria, CA). In some case, the slides were counterstained with hematoxylin. The slides were mounted with Depex (VWR International). Nissl staining was performed on brain section, according to standard protocol.

### Image acquisition

For all IHC and coloration, slices were acquired using the Hamamatsu slide scanner.

### DNA extraction

DNA was extracted from brain, spinal cord and peripheral organs using chloroform/phenol protocol.

### RNA extraction

Total RNA was extracted from a part of brain using Trizol or TriReagent (Sigma). One microgram of total RNA was transcribed into cDNA with Transcriptor First Strand cDNA synthesis kit (Roche) according the manufacturer's instructions.

### q-PCR

cDNA was amplified with SyberGreen (Roche). Primers for RT-qPCR were table above. The amplification protocol for all primers a hot start (95°C for 5 min), 45 amplification cycles (95°C for 15s, 60°C for 1 min) and a melt-curve analysis. Data were analyzed using the Lightcycler 480 software with efficiency factor for each gene and normalized to actine.

| Name | Primer 5' -> 3' |
|---|---|
| *Actine 126 For* | TCC TGA GCG CAA GTA CTC TGT |
| *Actine_127 Rev* | CTG ATC CAC ATC TGC TGG AAG |
| *Cyp46A1 mouse For* | GGC TAA GAA GTA TGG TCC TGT TGT AAG A |
| *cyp46A1 mouse Rev* | GGT GGA CAT CAG GAA CTT CTT GAC T |
| *ApoE For* | GTC ACA TTG CTG ACA GGA TGC CTA |
| *ApoE Rev* | GGG TTG GTT GCT TTG CCA CTC |
| *Hmgcr For* | CCC CAC ATT CAC TCT TGA CGC TCT |
| *Hmgcr Rev* | GCT GGC GGA CGC CTG ACA T |
| *Srebp1 For* | GGT CCA GCA GGT CCC AGT TGT |
| *Srebp1 Rev* | CTG CAG TCT TCA CGG TGG CTC |
| *Srebp2 For* | TGT TGA CGC AGA CAG CCA ATG |
| *cyp46A1human For* | CGA GTC CTG AGT CGG TTA AGA AGT T |
| *cyp46A1 human Rev* | AGT CTG GAG CGC ACG GTA CAT |
| *MBP For* | GCCTGTCCCTCAGCAGATT |
| *MBP_Rev* | CTTCAAACGAAAAGGGACGA |
| *Dhcr7 For* | AGACATTTGGGCCAAGACAC |
| *Dhcr7 Rev* | AACCTGGCAGAAATCTGTGG |
| *Dgat1 For* | CCTCAGCCTTCTTCCATGAG |
| *Dgat1 Rev* | ACTGGGGCATCGTAGTTGAG |
| *mADCK3 for* | CCA CCT CTC CTA TGG GCA GA |
| *mADCK3 rev* | CCG GGC CTT TTC AAT GTC T |

Vector Genome Copy Number was measured by qPCR on extracted genomic DNA from spinal cord, brain, sciatic nerve and peripheral organs using the Light Cycler 480 SYBR Green I Master (Roche, France). The results (vector genome copy number per cell) were expressed as n-fold differences in the transgene sequence copy number relative to the Adck3 gene copy as internal standard (number of viral genome copy for 2N genome).

### Cholesterol and oxysterol measurements

Cholesterol and oxysterol analysis followed the 'gold standard' method (Dzeletovic et al., 1995) to minimize the formation of autoxidation artefacts. Briefly, mouse brain tissue samples were weighed and homogenized with a TissueLyser II apparatus (Qiagen) in a 500 µl solution containing butylated hydroxytoluene (BHT, 50 µg/ml) and EDTA (0.5 M). At this point, a mix of internal standards was added [epicoprostanol, 2H7-7-lathosterol, 2H6-desmosterol, 2H6-lanosterol and 2H7-24(R/S)-hydroxycholesterol] (Avanti Polar Lipids). Alkaline hydrolysis was performed under Ar using 0.35 M ethanolic KOH for 2 h at room temperature. After neutralization of the solution with phosphoric acid, sterols were extracted in chloroform. The lower phase was collected, dried under a stream of nitrogen and the residue was dissolved in toluene. Oxysterols were then separated from the cholesterol and its precursors on a 100 mg Isolute silica cartridge (Biotage); cholesterol was eluted in 0.5% propan-2-ol in hexane followed by oxysterols in 30% propan-2-ol in hexane. The sterol and oxysterol fractions were independently silylated with Regisil^{®} + 10% TMCS [bis(trimethylsilyl) trifluoro-acetamide + 10% trimethylchlorosilane] (Regis technologies) as described previously (Chevy et al., 2005). The trimethylsilylether derivatives of sterols and oxysterols were separated by gas chromatography (Hewlett-Packard 6890 series) in a medium polarity capillary column RTX-65 (65% diphenyl 35% dimethyl polysiloxane, length 30 m, diameter 0.32 mm, film thickness 0.25 µm; Restesk). The mass spectrometer (Agilent 5975 inert XL) in series with the gas chromatography was set up for detection of positive ions. Ions were produced in the electron impact mode at 70 eV. They were identified by the fragmentogram in the scanning mode and quantified by selective monitoring of the specific ions after normalization and calibration with the appropriate internal and external standards [epicoprostanol m/z 370, 2H7-7-lathosterol m/z 465, 2H6-desmosterol m/z 358, 2H6-lanosterol m/z 504, 2H7-24(R/S)-hydroxycholesterol m/z 553, cholesterol m/z 329, 7-lathosterol m/z, 7-dehydrocholesterol m/z 325, 8-dehydrocholesterol m/z 325, desmosterol m/z 343, lanosterol m/z 393 and 24(R/S)-hydroxycholesterol m/z 413].

### Western Blot analysis

Total proteins were extracted from several regions of 45days old MECP2 -/y males. Total protein concentrations were determined using the BCA kit (Pierce). Equal amounts of total protein extract (30 µg) were electrophoretically separated using SDS-PAGE in 4-12% Bis-Tris gels (NuPAGE^{®} Novex Bis-tris midi gel 15 or 26 wells, Life Technologies, Carlsbad, USA) and transferred to nitrocellulose membranes. Blocked membranes (5% non-fat dry milk in TBS-0.1% Tween-20) were incubated with primary antibodies overnight at 4°C, and washed three times with TBS-0.1% Tween-20 (T-BST) for 10 min. Membranes were then labeled with secondary IgG-HRP antibodies raised against each corresponding primary antibody. After three washes with T-BST, the membranes were incubated with ECL chemiluminescent reagent (Clarity Western ECL substrate; GE Healthcare, Little Chalfont, UK) according to the instructions of the supplier. Peroxydase activity was detected with camera system Fusion TX7 (Fisher Scientific). Normalization was done by densitometry analysis with the Quantity One 1D image analysis software (version 4.4; Biorad, Hercules, CA, USA). The optical densities were normalized with respect to a "standard protein" (GAPDH). A partition ratio was calculated and normalized with respect to the sample with the highest value defined as 1.

### Satistical analysis

Statistical analysis was performed using unpaired Student's t-test and 2 way ANOVA. Results are expressed as mean ± SEM. Significant thresholds were set at P<0.05, P<0.01 and P<0.001, as defined in the text. All analyses were performed using GraphPad Prism (GraphPad Software, La Jolla, USA).

### RESULTS

### Decreased levels of CYP46A1 protein levels in several brain area of C57BL6/Mecp2^{tm1.1Bird} mouse model of Rett syndrome

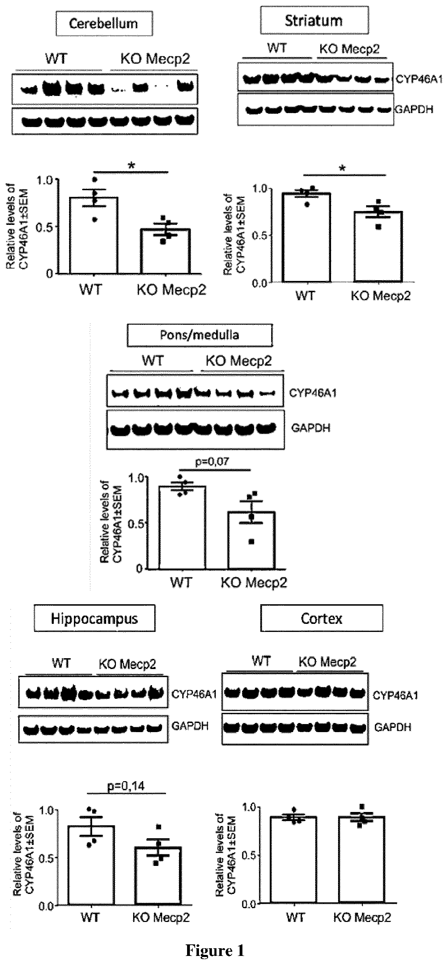

The levels of CYP46A1 were evaluated in frozen biopsies from 4 MECP2 ^{-/y} animals at 45 days of age. In particular, CYP46A1 protein levels were analyzed in the cerebellum, pons, striatum, hippocampus and cortex of affected animals and compared to WT littermates (Figure 1). Western-blot analysis demonstrated a significant reduction in CYP46A1 protein levels in the cerebellum, striatum and a trend has been observed in the pons and hippocampus and no differences in the cortex relatively to control littermates (Figure 1).

Overall, these data support the relevance of CYP46A1, a key enzyme in cholesterol metabolism pathway, as a therapeutic target in human Rett Syndrome.

### Deregulation of the cholesterol metabolism pathway in the brain of C57BL6/Mecp2^{tm1.1Bird} model of Rett syndrome

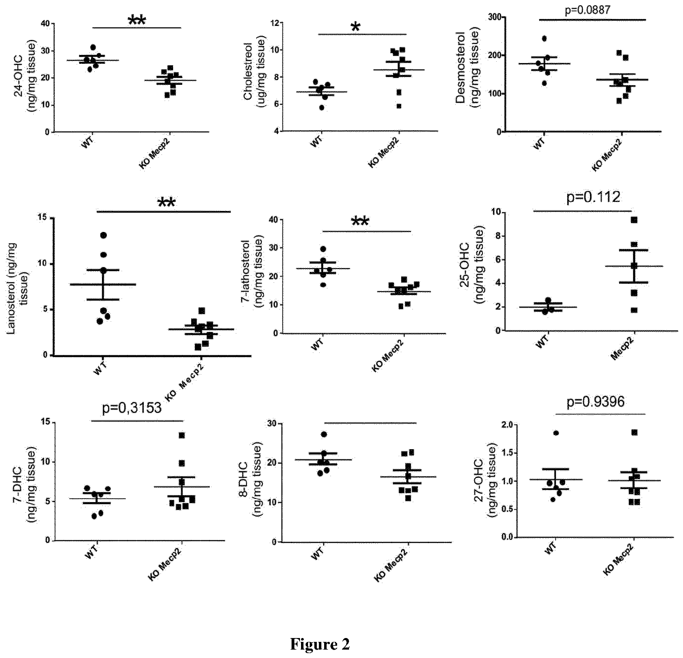

Previously, alteration of cholesterol and overall lipid change in Rett syndrome have been reported (Lütjohann et al. 2018b). To determine the impact of CYP46A1 deficiency in Rett syndrome, the cholesterol metabolism was analyzed by performing quantitative measurements of both sterols and oxysterols by gas-chromatography mass spectrometry (GC/MS) in total brain extracts from 4 MECP2 ^{-/y} animals at 45 days and age-matched littermates. Therefore, lanosterol, the first sterol of the cholesterol metabolic pathway, along with cholesterol intermediates from the Kandutsch-Russell (lathosterol) and Bloch (desmosterol) pathways were measured. The cholesterol and 24-hydroxycholesterol (24S-OHC) content were first compared and a statistically significant reduction was observed in the levels of 24S-OHC in MECP2 KO mice relatively to wild-type littermates (Figure 2) and a significant increase was observed for the cholesterol. A significant decrease of the lanosterol and 7-lathosterol was also measured in the brain of KO MECP2 compare to WT littermates. Finally, a trend to decrease but not significant was measured for desmosterol and 8DHC (Figure 2). No statistically significant differences were found for the oxysterols 25S-OHC and 27S-OHC and for the remaining sterols (7-DHC) (Figure 2).

Altogether, these results suggest alterations in the cholesterol metabolism pathway in Rett syndrome affected regions.

### CYP46A1 overexpression in the mild mouse model of Rett syndrome could alleviate the motor impairment associated to the Rett syndrome and improve histological and molecular phenotype associated with Rett syndrome

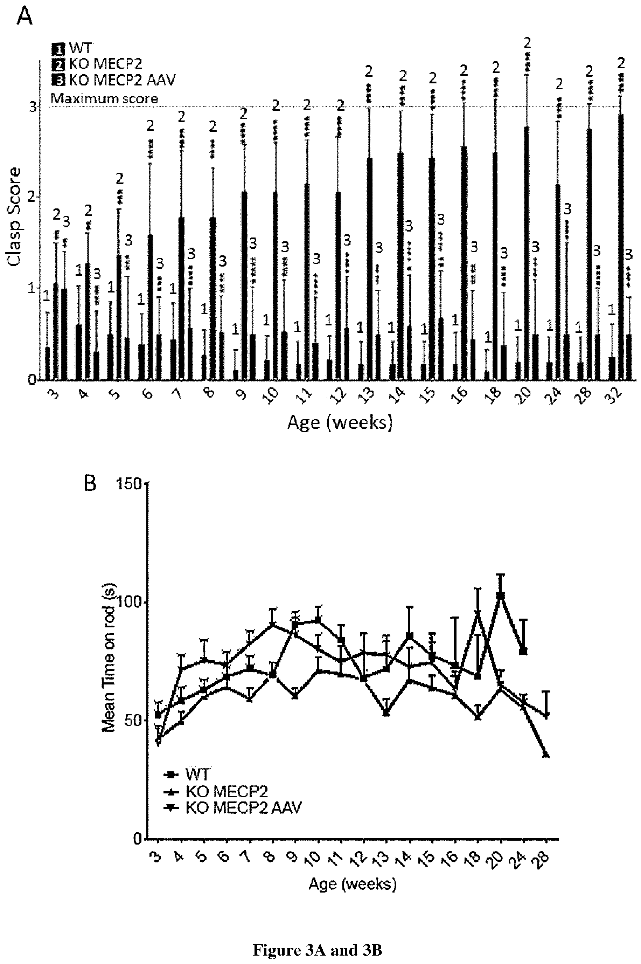

The inventors investigated whether the upregulation of the cholesterol metabolism pathway, through increase in the levels of CYP46A1, could improve motor alteration in an in vivo model of Rett Syndrome: the *mild KO MECP2* model, especially the male KO that are more severely affected. Overexpression of CYP46A1 by intravenous administration of AAVPHP.eB-CYP46A1-HA in 3 weeks old MECP2 ^{-/y} mice clearly alleviate motor alteration in the mouse model. It significantly corrects motor impairment measure by clasping test (Figure 3A) and rotarod (Figure 3B) for 29 weeks post-injection (maximum time of investigation).

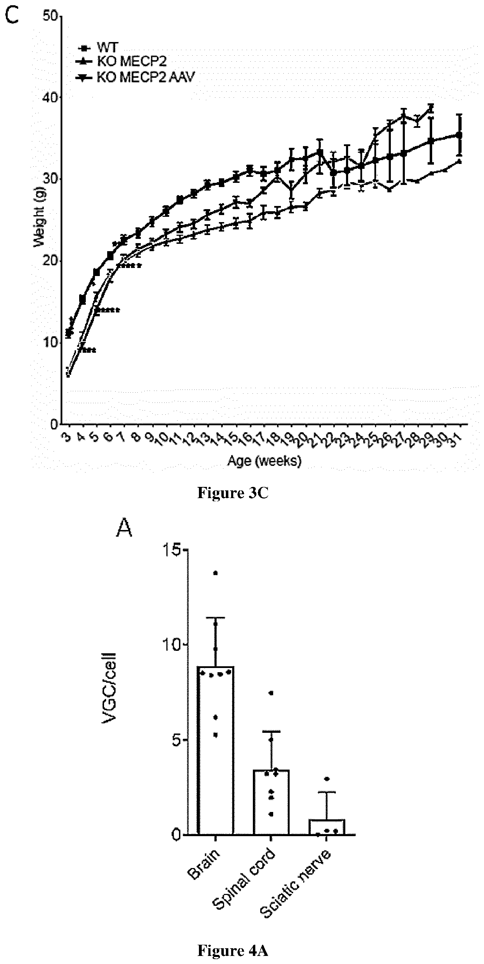

CYP46A1 overexpression also significantly leads to a better growth of the mice and restores a normal body weight of mice compared to non-treated animals. Treated animals have a similar growth of WT littermates (Figure 3C).

A group of mice have been euthanized at 16 weeks of age to evaluate histological and molecular rescue and the rest of the cohort have been maintained until 32 weeks of age.

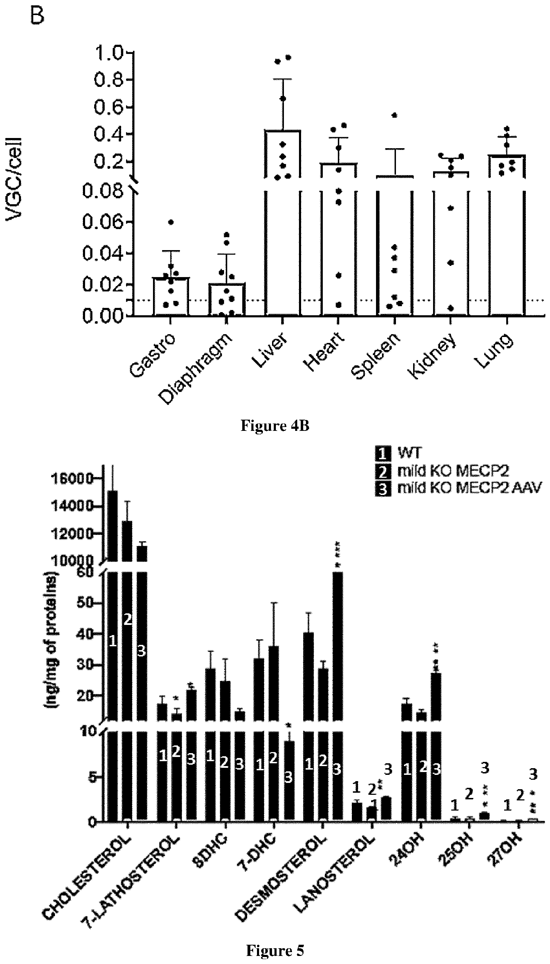

Biodistribution study revealed around 8 vector genome copy (VGC) in the brain (Figure 4A), around 4 VGC in the spinal cord and 1VGC in the sciatic nerve, as well as a really low transduction of the peripheral tissues with a maximum of 0.4 VGC in the liver and less that 0.2 VGC in the other peripheral organs (Figure 4B).

This biodistribution perfectly correlates with a large expression of CYP46A1-HA in all structure of the brain, especially in cortex, striatum, hippocampus and cerebellum (data not shown) , that also confirmed the results previously obtained in WT animals (non-presented data).

Target engagement has been validated with the quantification of 24 hydroxycholesterol in the lumbar spinal cord of the animals, revealing a 1.9 fold increase in treated animals compared to non-treated animals (Figure 5). In addition, the inventors reported an overall elevation of the cholesterol pathway with increased levels of 7-lathosterol, lanosterol, 25-OH and 27-OH cholesterol compare to non-treated mild KO MECP2 males (Figure 5).

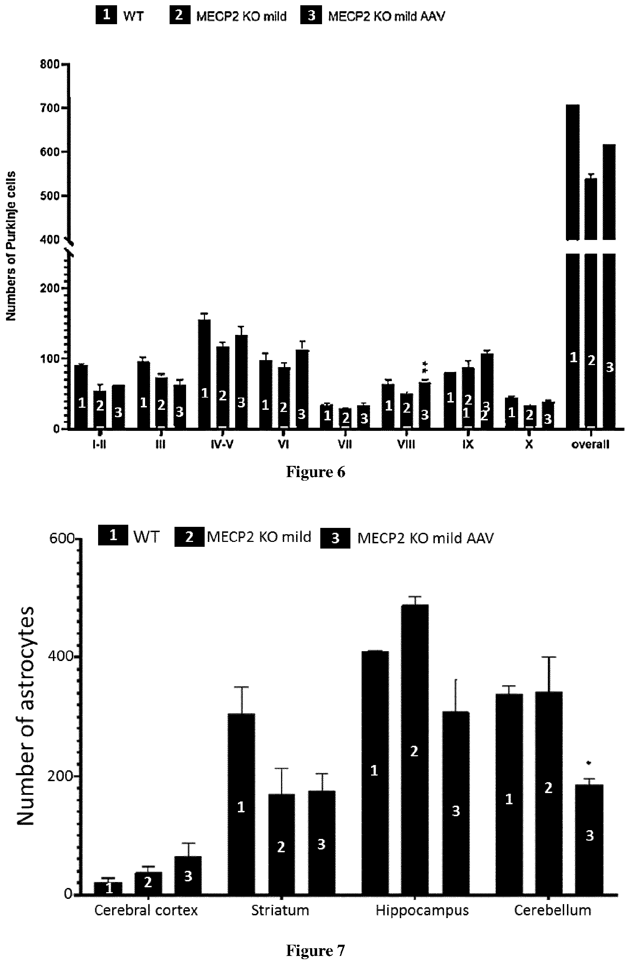

A partial preservation of the Purkinje cells (PCs) has been demonstrated in the cerebellum with an overall improvement that can also be observed in the different lobules, with a pvalue< 0.0001 (effect on lobule and on treatment with ANOVA test) (Figure 6).

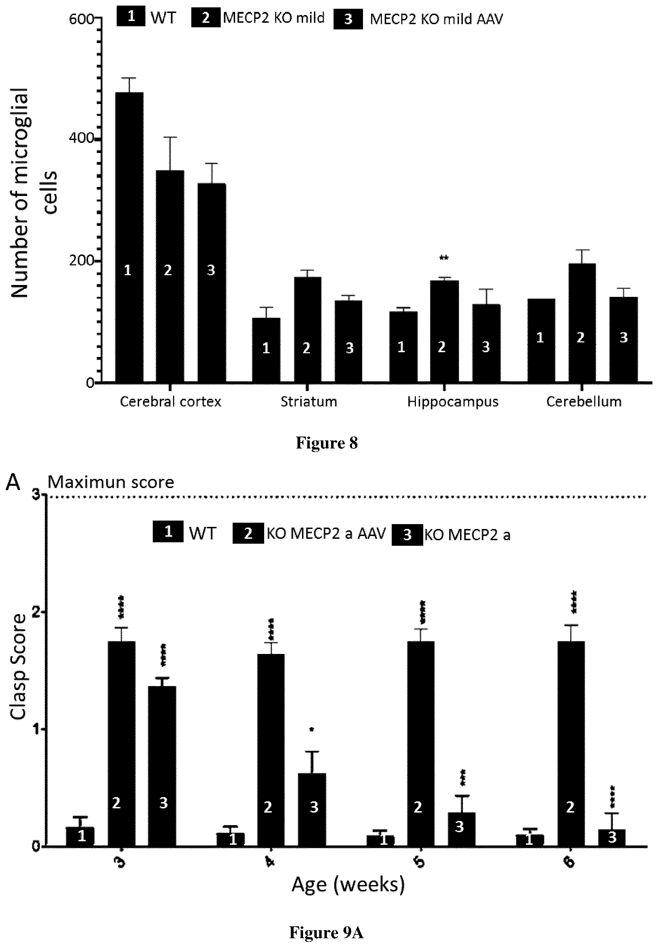

The inventors then studied the effect of the treatment on the neuroinflammation. The inventors demonstrated an improvement of astrogliosis, especially in hippocampus in the treated animal compare to non-treated animals (Figure 7). In addition, an improvement of microgliosis, especially in striatum, hippocampus and cerebellum of treated animals compare to non-treated animals (Figure 8). Overall, an important finding is also the fact that AAVPHP.eB delivery did not lead to any long-term inflammation in the brain (Figures 7 and 8).

### CYP46A1 overexpression alleviates behavioral alterations in an aggravated male model of Rett syndrome and improve phenotype associated with Rett syndrome.

Based on treatment in mildly affected animals, investigations were performed to determine whether the upregulation of the cholesterol metabolism pathway, through increase in the levels of CYP46A1, could improve motor alteration in a more severe *in vivo* model of Rett syndrome: the aggravated KO MECP2.

Overexpression of CYP46A1 by intravenous administration of AAVPHP.eB-CYP46A1-HA in 3 weeks old mice clearly alleviate motor alteration in the aggravated mouse model.

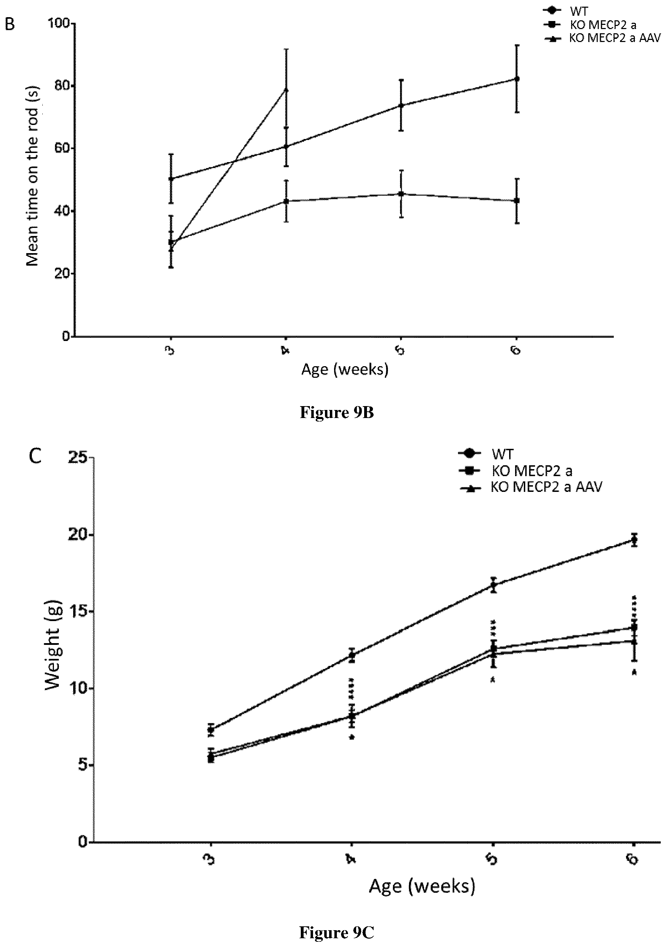

**AAVPHP.eB-CYP46A1** administration clearly decreases motor impairment measure by clasping test (Figure 9A) and alleviate alteration measured by rotarod (Figure 9B) evaluation until 4 weeks and no longer due to the confinement of the COVID19 (experiment ongoing). KO mice have an impaired growth but the 3 weeks of treatment did not lead to any improvement (Figure 9C).

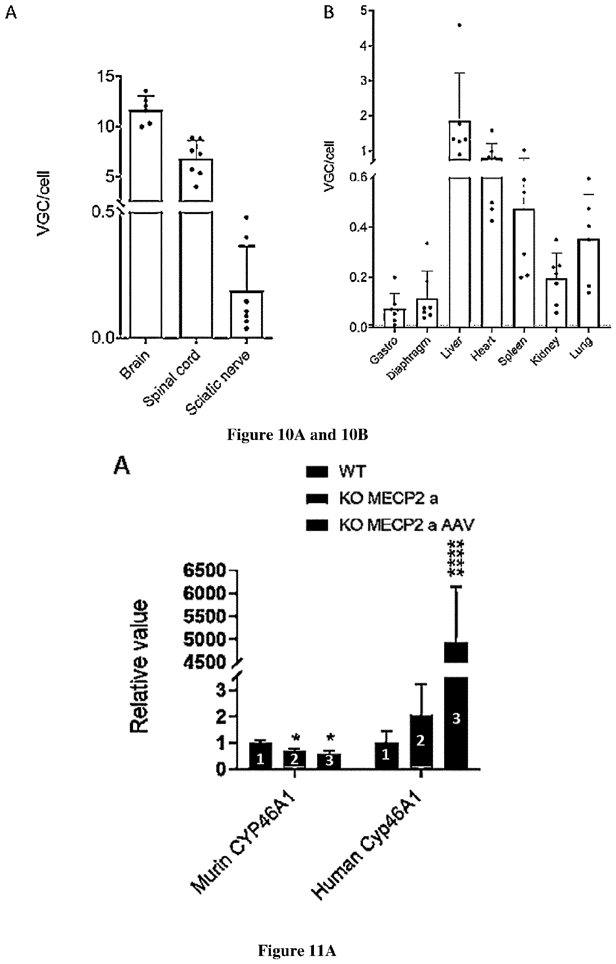

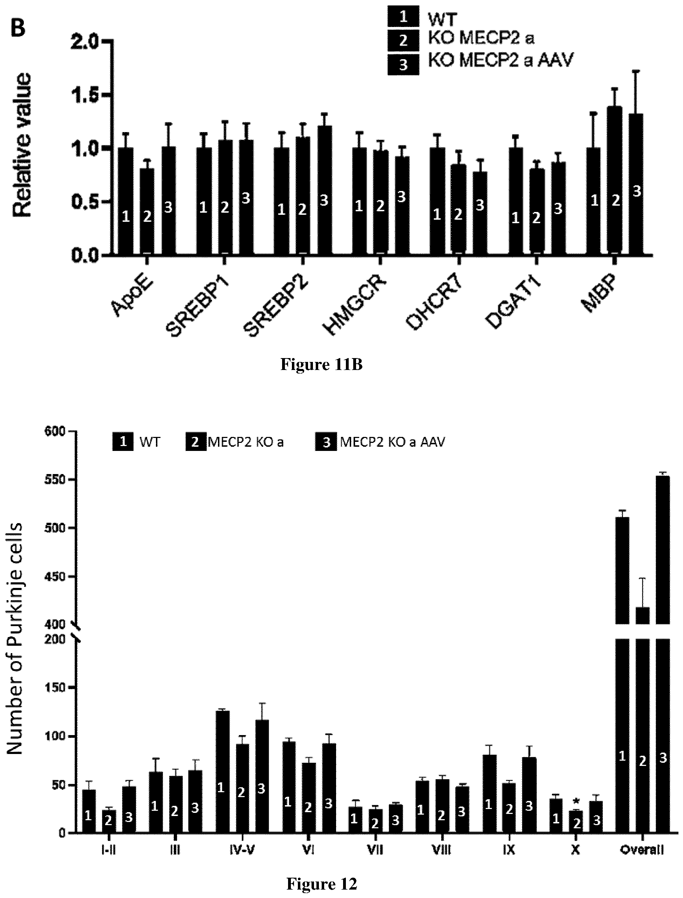

Mice were euthanized at 6 weeks of age (40 days) for histological and molecular analysis. Biodistribution study, revealed around 12 vector genome copy (VGC) in the brain (Figure 10A) and around 7 VGC in the spinal cord (Figure 10A) as well as a low transduction of the peripheral tissues with a maximum of 2 VGC in the liver, 1VGC in the heart and less that 0.5 VGC in the other peripheral organs (Figure 10B) only 20 days after injection. This biodistribution correlated with a large expression of hCYP46A1 mRNA (Figure 11A), without affection endogenous murine levels (Figure 11A). Regarding other genes involved in the cholesterol pathway, the inventors reported a trend to normalization of ApoE levels in treated animals compare to non-treated animals (Figure 11B). None of the other genes were impacted at 40 days of age but need to be evaluated later on in the cohort of mice for survival.

A preservation of the Purkinje cells (PCs) has been demonstrated in the cerebellum with an overall improvement that can also be observed in the different lobules, with a p value < 0.0001 (effect on lobule and on treatment with ANOVA test) (Figure 12).

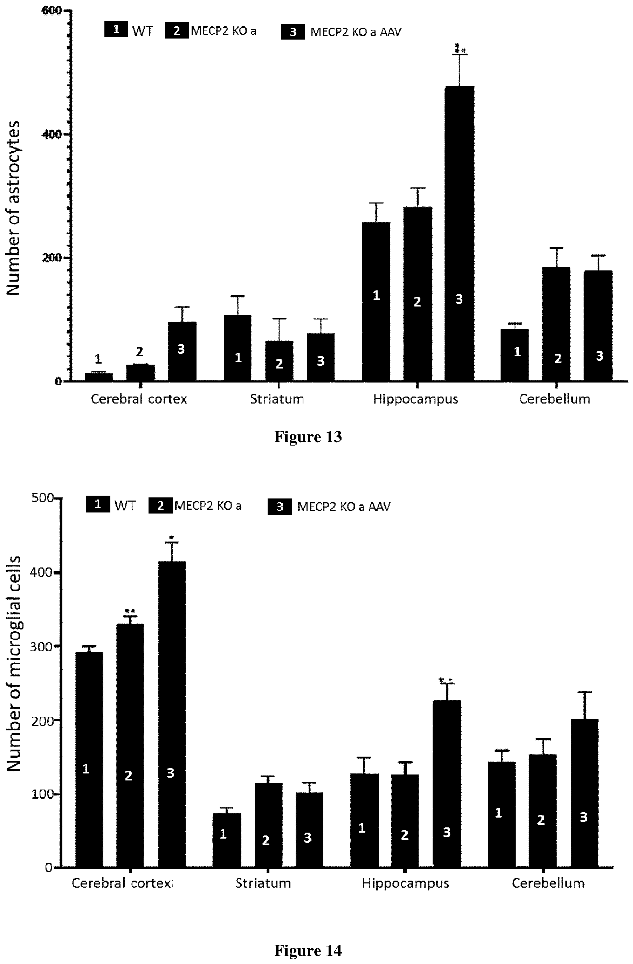

The inventors then studied the effect of the treatment on the neuroinflammation. The inventors demonstrated no major effect on astrogliosis, except an increase in cerebral cortex (Figure 14) and hippocampus. For both astrogliosis and microgliosis, no major difference were observed at this age in aggravated KO MECP2 males and further investigations need to be done at later stage. Another cohort of treated animals is ongoing to evaluate long-term improvement and effect on survival.

### CYP46A1 overexpression alleviates behavioral alterations in an aggravated female model of Rett syndrome.

As Rett syndrome affect females in Humans, the inventor wanted to investigate the effect of CYP46A1 overexpression in aggravated KO MECP2 females.

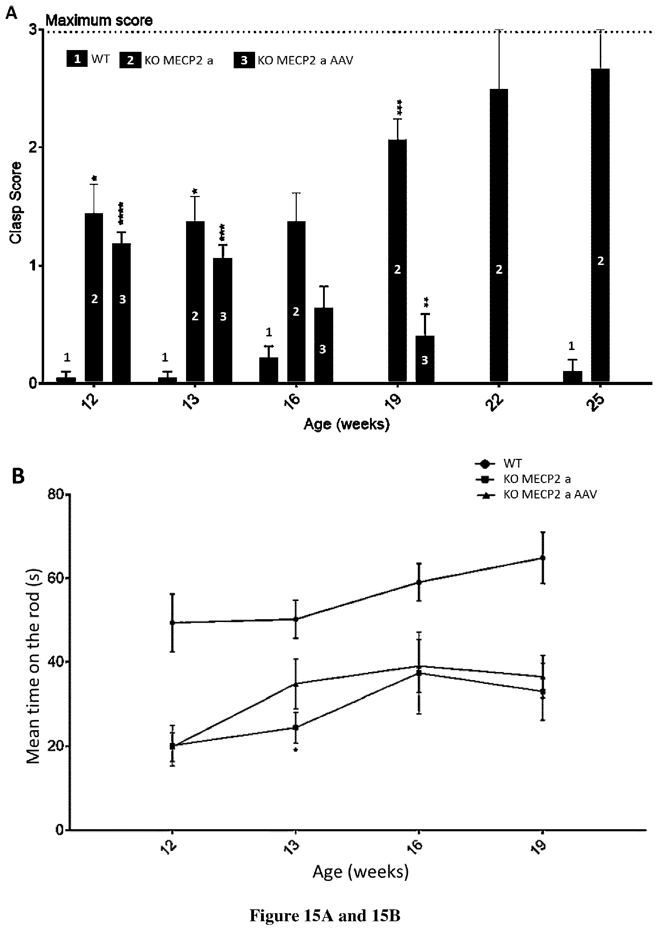

AAVPHP.eB-CYP46A1 administration clearly and significantly decreases motor impairment measure by clasping test (Figure 15A) and alleviate alteration measured by rotarod (Figure 15B) evaluation until 19 weeks and no longer due to the confinement of the COVID19 (experiment ongoing).

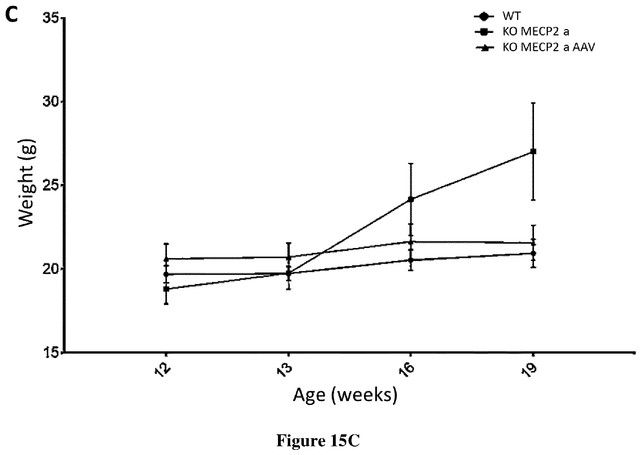

KO mice have an impaired growth with trend to obesity and a clear improvement of this phenotype was observed in the treated animals (Figure 15C).

Altogether these data suggest that CYP46A1 overexpression alleviates behavioral alterations in a mild and an aggravated model of Rett syndrome and both in male and female and is a therapeutic target.

### REFERENCES:

Throughout this application, various references describe the state of the art to which this invention pertains.
Amir, Ruthie E. et al. 1999. "Rett Syndrome Is Caused by Mutations in X-Linked MECP2, Encoding Methyl-CpG-Binding Protein 2." Nature Genetics 23(2): 185-88. http://www.ncbi.nlm.nih.gov/pubmed/10508514 (June 22, 2018).
Armstrong, D D. 1997. "Review of Rett Syndrome." Journal of neuropathology and experimental neurology 56(8): 843-49. http://www.ncbi.nlm.nih.gov/pubmed/9258253 (June 22, 2018).
Buchovecky, Christie M et al. 2013. "A Suppressor Screen in Mecp2 Mutant Mice Implicates Cholesterol Metabolism in Rett Syndrome." Nature Genetics 45(9): 1013-20. http://www.ncbi.nlm.nih.gov/pubmed/23892605 (March 4, 2019).
Duncan Armstrong, Dawna. 2005. "Neuropathology of Rett Syndrome." Journal of Child Neurology 20(9): 747-53. http://www.ncbi.nlm.nih.gov/pubmed/16225830 (March 4, 2019).
Gadalla, Kamal K.E. et al. 2017. "Development of a Novel AAV Gene Therapy Cassette with Improved Safety Features and Efficacy in a Mouse Model of Rett Syndrome." Molecular Therapy - Methods & Clinical Development 5: 180-90. http://www.ncbi.nlm.nih.gov/pubmed/28497075 (June 22, 2018).
Gadalla, Kamal KE et al. 2013. "Improved Survival and Reduced Phenotypic Severity Following AAV9/MECP2 Gene Transfer to Neonatal and Juvenile Male Mecp2 Knockout Mice." Molecular Therapy 21(1): 18-30. http://www.ncbi.nlm.nih.gov/pubmed/23011033 (June 22, 2018).
Garg, S. K. et al. 2013. "Systemic Delivery of MeCP2 Rescues Behavioral and Cellular Deficits in Female Mouse Models of Rett Syndrome." Journal of Neuroscience 33(34): 13612-20. http://www.ncbi.nlm.nih.gov/pubmed/23966684 (June 22, 2018).
Guy, Jacky et al. 2001. "A Mouse Mecp2-Null Mutation Causes Neurological Symptoms That Mimic Rett Syndrome." Nature Genetics 27(3): 322-26. http://www.ncbi.nlm.nih.gov/pubmed/11242117 (June 22, 2018).
Hagberg, B et al. 1985. "Rett Syndrome: Criteria for Inclusion and Exclusion." Brain & development 7(3): 372-73. http://www.ncbi.nlm.nih.gov/pubmed/4061772 (June 22, 2018).
Lütjohann, Dieter et al. 2018a. "Identification of Correlative Shifts in Indices of Brain Cholesterol Metabolism in the C57BL6/Mecp2 Tm1.1Bird Mouse, a Model for Rett Syndrome." Lipids 53(4): 363-73. http://www.ncbi.nlm.nih.gov/pubmed/29770459 (March 4, 2019)
Lütjohann, Dieter et al. 2018b. "Identification of Correlative Shifts in Indices of Brain Cholesterol Metabolism in the C57BL6/Mecp2 Tm1.1Bird Mouse, a Model for Rett Syndrome." Lipids 53(4): 363-73. http://www.ncbi.nlm.nih.gov/pubmed/29770459 (May 3, 2019).
Na, Elisa S, Erika D Nelson, Ege T Kavalali, and Lisa M Monteggia. 2013. "The Impact of MeCP2 Loss- or Gain-of-Function on Synaptic Plasticity." Neuropsychopharmacology 38(1): 212-19. http://www.ncbi.nlm.nih.gov/pubmed/22781840 (June 22, 2018).
Nagy, Gabor, and Susan L Ackerman. 2013. "Cholesterol Metabolism and Rett Syndrome Pathogenesis." Nature Genetics 45(9): 965-67. http://www.ncbi.nlm.nih.gov/pubmed/23985682 (March 4, 2019).

## Claims

1. A vector for use in the treatment of Rett syndrome (RTT) which vector comprises cholesterol 24-hydroxylase encoding nucleic acid.

2. The vector for use in the treatment of RTT according to claim 1, wherein the RTT is associated with at least one autism spectrum disorder.

3. The vector for use in the treatment of RTT according to claim 2, wherein the RTT is associated with intellectual disability.

4. The vector for use in the treatment of RTT according to any one of claims 1 to 3, comprising a nucleic acid sequence that encodes the amino acid sequence SEQ ID N°2.

5. The vector for use in the treatment of RTT according to any one of claims 1 to 4, comprising the nucleic acid sequence SEQ ID N°1 or a variant thereof exhibiting a nucleotide sequence identity of at least 75% with SEQ ID N°1.

6. The vector for use in the treatment of RTT according to any one of claims 1 to 5, which is selected from the group of adenovirus, lentivirus, retrovirus, herpesvirus and Adeno-Associated Virus (AAV) vectors.

7. The vector for use in the treatment of RTT according to any one of claims 1 to 6, which is an AAV vector.

8. The vector for use in the treatment of RTT of claim 7, which is an AAV9, AAV10 vector, such as AAVrh.10, or AAVPHP.eB, preferably an AAVPHP.eB.

9. The vector for use in the treatment of RTT according to any one of claims 1 to 8, which is to be administered intravenously.

10. The vector for use in the treatment of RTT according to any one of claims 1 to 9, which is to be administered directly into the brain or cerebrospinal fluid of the patient.

11. The vector for use in the treatment of RTT of claim 10, which is to be administered to neurons (cortical, hippocampal, striatal and cerebellar).

12. The vector for use in the treatment of RTT according to any one of claims 1 to 11, which is to be administered by intravascular, intravenous, intranasal, intraventricular, retroorbital or intrathecal injection.

13. A pharmaceutical composition for use in the treatment of Rett syndrome, which comprises a therapeutically effective amount of a vector as defined in any one of claims 1 to 12.

14. The vector for use in the treatment of RTT according to claim 1, wherein the treatment comprises delivering said vector to the brain and/or spinal cord of the subject, whereby said vector transduces cells in the brain and/or spinal cord, and whereby cholesterol 24-hydroxylase is expressed by the transduced cells at a therapeutically effective level.

## Patentansprüche

1. Ein Vektor zur Verwendung in der Behandlung des Rett-Syndroms (RTT), wobei der Vektor Cholesterin-24-Hydrolase-kodierende Nukleinsäure umfasst.

2. Der Vektor zur Verwendung in der Behandlung von RTT nach Anspruch 1, wobei das RTT mit mindestens einer Autismus-Spektrum-Störung assoziiert ist.

3. Der Vektor zur Verwendung in der Behandlung von RTT nach Anspruch 2, wobei das RTT mit geistiger Behinderung assoziiert ist.

4. Der Vektor zur Verwendung in der Behandlung von RTT nach einem der Ansprüche 1 bis 3 umfassend eine Nukleinsäuresequenz, die die Aminosäuresequenz SEQ ID NO: 2 kodiert.

5. Der Vektor zur Verwendung in der Behandlung von RTT nach einem der Ansprüche 1 bis 4 umfassend die Nukleinsäuresequenz SEQ ID NO:1 oder eine Variante davon, die eine Nukleotidsequenzidentität von mindestens 75% mit SEQ ID NO:1 aufweist.

6. Der Vektor zur Verwendung in der Behandlung von RTT nach einem der Ansprüche 1 bis 5, der ausgewählt ist aus der Gruppe aus Adenovirus-, Lentivirus-, Retrovirus-, Herpesvirus- und Adeno-assoziierte Virus (AAV) Vektoren.

7. Der Vektor zur Verwendung in der Behandlung von RTT nach einem der Ansprüche 1 bis 6, der ein AAV Vektor ist.

8. Der Vektor zur Verwendung in der Behandlung von RTT nach Anspruch 7, der ein AAV9, AAV10 Vektor, wie AAVrh.10 oder AAVPHP.eB, bevorzugt ein AAVPHP.eB ist.

9. Der Vektor zur Verwendung in der Behandlung von RTT nach einem der Ansprüche 1 bis 8, der intravenös zu verabreichen ist.

10. Der Vektor zur Verwendung in der Behandlung von RTT nach einem der Ansprüche 1 bis 9, der direkt ins Gehirn oder die Zerebrospinalflüssigkeit des Patienten zu verabreichen ist.

11. Der Vektor zur Verwendung in der Behandlung von RTT nach einem der Ansprüche 1 bis 10, der in Neurone (kortikal, hippocampal, striatal und zerebellar) zu verabreichen ist.

12. Der Vektor zur Verwendung in der Behandlung von RTT nach einem der Ansprüche 1 bis 11, der mittels intravaskulärer, intravenöser, intranasaler, intraventrikulärer, retroorbitaler oder intrathekaler Injektion zu verabreichen ist.

13. Eine pharmazeutische Zusammensetzung zur Verwendung in der Behandlung des Rett-Syndroms, die eine therapeutisch wirksame Menge eines Vektors wie in einem der Ansprüche 1 bis 12 definiert, umfasst.

14. Der Vektor zur Verwendung in der Behandlung von RTT nach Anspruch 1, wobei die Behandlung das Liefern des Vektors an das Gehirn und/oder das Rückenmark des Subjekts umfasst, wobei der Vektor Zellen im Gehirn und/oder dem Rückenmark transduziert und wobei durch die transduzierten Zellen Cholesterin-24-Hydrolase auf einem therapeutisch wirksamen Level exprimiert wird.

## Revendications

1. Vecteur pour une utilisation dans le traitement du syndrome de Rett (RTT) lequel vecteur comprend une séquence d'acide nucléique codant pour la cholestérol 24-hydroxylase.

2. Vecteur pour une utilisation dans le traitement du RTT selon la revendication 1, dans lequel le RTT est associé avec au moins un trouble du spectre autistique.

3. Vecteur pour une utilisation dans le traitement du RTT selon la revendication 2, dans lequel le RTT est associé à une déficience intellectuelle.

4. Vecteur pour une utilisation dans le traitement du RTT selon l'une quelconque des revendications 1 à 3, comprenant une séquence d'acide nucléique codant pour la séquence d'acides aminés SEQ ID N°2.

5. Vecteur pour une utilisation dans le traitement du RTT selon l'une quelconque des revendications 1 à 4, comprenant la séquence d'acide nucléique SEQ ID N°1 ou une variante de celle-ci présentant une identité de séquence nucléotidique d'au moins 75% avec SEQ ID N°1.

6. Vecteur pour une utilisation dans le traitement du RTT selon l'une quelconque des revendications 1 à 5, qui est sélectionné dans le groupe des vecteurs des adénovirus, lentivirus, rétrovirus, herpèsvirus et les virus adénoassociés (AAV)

7. Vecteur pour une utilisation dans le traitement du RTT selon l'une quelconque des revendications 1 à 6, qui est un vecteur AAV.

8. Vecteur pour une utilisation dans le traitement du RTT selon la revendication 7, qui est un vecteur AAV9, AAV10, tel que AAVrh.10, ou AAVPHP.eB, de préférence un AAVPHP.eB.

9. Vecteur pour une utilisation dans le traitement du RTT selon l'une quelconque des revendications 1 à 8, qui est destiné à être administré en intraveineuse.

10. Vecteur pour une utilisation dans le traitement du RTT selon l'une quelconque des revendications 1 à 9, qui est destiné à être administré directement dans le cerveau ou le fluide cérébrospinal du patient.

11. Vecteur pour une utilisation dans le traitement du RTT selon la revendication 10, qui est destiné à être administré aux neurones (corticaux, hippocampiques, striataux et cérébelleux).

12. Vecteur pour une utilisation dans le traitement du RTT selon l'une quelconque des revendications 1 à 11, qui est destiné à être administré par injection intravasculaire, intraveineuse, intranasale, intraventriculaire, rétro-orbitaire ou intrathécale.

13. Composition pharmaceutique pour une utilisation dans le traitement du syndrome de Rett, qui comprend une quantité thérapeutiquement efficace d'un vecteur tel que défini dans l'une des quelconques revendications 1 à 12.

14. Vecteur pour une utilisation dans le traitement du RTT selon la revendication 1, dans lequel le traitement comprend la délivrance dudit vecteur au cerveau et/ou à la moelle épinière du sujet, lequel vecteur transduit des cellules dans le cerveau et/ou dans la moelle épinière, et où la cholestérol 24-hydroxylase est exprimée par les cellules transduites à un niveau thérapeutiquement efficace.
